# FASCICULE DE BREVET EUROPEEN

(11) **EP 3 791 911 B1**
(45) Date de publication et mention de la délivrance du brevet: **21.02.2024**
(21) Numéro de dépôt: 20183095.7
(22) Date de dépôt: 30.06.2020
(51) Int. Cl.: A61M 16/00

(54) **VENTILATEUR MÉDICAL PROTÉGÉ PAR UNE STRUCTURE-EXOSQUELETTE**
MEDIZINISCHES BEATMUNGSGERÄT, DAS DURCH EINE EXOSKELETT-STRUKTUR GESCHÜTZT IST
MEDICAL VENTILATOR PROTECTED BY AN EXOSKELETON STRUCTURE

(30) Priorité: 11.09.2019 FR 1909987
(43) Date de publication de la demande: 17.03.2021
(73) Titulaire: Air Liquide Medical Systems, 92160 Antony (FR)
(72) Inventeur: GIARD, Pauline, 92160 Antony (FR); ANDRE DIAS, Sofia, 92160 Antony (FR); GERMANI, Damien, 92320 Chatillon (FR); HARANT, Catherine, 92160 Antony (FR); LEBATTEUR, Nicolas, 92160 Antony (FR); LOPEZ, Julien, 92160 Antony (FR); RUSSO, Leslie, 92160 Antony (FR)
(74) Mandataire: Air Liquide

(56) Documents cités:
- WO-A1-2014/145253
- CN-A- 109 157 362
- FR-A3- 3 076 223

## Description

L'invention porte sur un ventilateur médical protégé par une structure-exosquelette agencée autour de la carcasse du ventilateur médical de manière à le protéger contre les chocs ou autres, en particulier un ventilateur adapté aux interventions d'urgence et au transport.

Afin d'assister certaines personnes ou patients dans leur fonction respiratoire, on utilise habituellement un appareil d'assistance respiratoire, aussi appelé « ventilateur médical », comprenant une micro-soufflante motorisée délivrant un gaz respiratoire, tel de l'air ou de l'air enrichi en oxygène, à débit non nul et/ou à une pression supérieure à la pression atmosphérique (> 1 atm).

La micro-soufflante motorisée, aussi appelée « compresseur » ou « turbine », aspire de l'air ambiant et le délivre à une pression donnée au patient. L'aspiration de l'air par la micro-soufflante, pendant le fonctionnement de son moteur, se fait grâce à une ou plusieurs roues à ailettes agencées sur un arbre ou axe rotatif entraîné en rotation par un moteur électrique, la (ou les) roue(s) à ailettes étant mobile(s) en rotation dans le compartiment interne de la volute de la micro-soufflante. L'air peut être enrichi en oxygène, c'est-à-dire additionné d'oxygène supplémentaire.

Les documents EP-A-2165078, EP-A-2102504, WO-A-2012/139681, US-A-2008/304986 et WO-A-2013/020167 décrivent de tels ventilateurs médicaux.

Certains ventilateurs dits « d'urgence-transport » sont destinés à être transportés sur le terrain par des secouristes civils ou militaires, et utilisés lors d'interventions d'urgence à l'extérieur, par exemple en cas d'accident, de catastrophe naturelle, de conflit, d'attaques chimiques ou biologique, ou autres.

Ainsi, un ventilateur d'urgence-transport doit pouvoir être utilisé dans une ambulance terrestre ou aérienne, i.e. un hélicoptère par exemple, être monté sur une station murale de rechargement ou accroché à un brancard ou à une paroi, posé sur une surface, notamment le sol. De même, il doit aussi pouvoir être utilisé en extérieur, y compris en conditions difficiles, notamment en présence de sable, neige, boue, milieu salin, températures extrêmes...

Les ventilateurs d'urgence-transport sont donc amenés à être soumis à de fortes contraintes d'utilisation, en particulier à des chocs, des chutes, des vibrations ou d'autres agressions extérieures.

Pour les protéger, on utilise couramment un sac ou sacoche de transport en matériau souple (e.g. tissu, tissé en polymère...), dans lequel le ventilateur médical est placé et servant à le protéger. Or, on comprend aisément que cette solution n'est pas idéale car les sacs de transport ne permettent pas de protéger efficacement le ventilateur contre les chocs importants, les chutes ou autres agressions. De plus, la présence du sac autour du ventilateur complique l'accès aux embouts de raccordement, aux connectiques, à l'IHM (i.e. interface homme-machine) ou à d'autres éléments, peut par ailleurs rendre difficile l'accrochage du ventilateur à certains supports muraux, notamment dans les véhicules de secours (e.g. ambulances, hélicoptères...) et nuit au refroidissement du ventilateur médical.

Par ailleurs, FR-A-3076223 propose un bâti-support formé de tubes portant un ventilateur médical, un compartiment à masque, une bouteille de gaz et d'autres éléments. Le transport de l'ensemble se fait au moyen de deux arceaux latéraux situés aux extrémités du bâti.

WO-A-2014/145253 enseigne un dispositif d'urgence de transport d'enfant. Là encore, le transport du dispositif se fait au moyen de deux arceaux latéraux.

De tels ensembles sont lourds, encombrants et n'assure qu'une protection relative et imparfaite.

Le problème qui se pose est de pouvoir protéger efficacement un ventilateur médical d'urgence-transport contre les fortes contraintes d'utilisation, en particulier les chocs, les chutes, les vibrations ou d'autres agressions extérieures, auxquelles il peut être soumis pendant son transport ou son utilisation, en particulier lors d'interventions d'urgence à l'extérieur, par exemple en cas d'accident, de catastrophe naturelle, de conflit ou autre, tout en permettant une manipulation aisée du ventilateur et sans gêner ou entraver les accès aux embouts de raccordement, aux connectiques, à l'IHM ou à d'autres éléments du ventilateur. De plus, le ventilateur médical doit être facile à transporter.

La solution de l'invention est alors un ventilateur médical, en particulier un ventilateur médical d'urgence-transport, comprenant une carcasse externe et une structure-exosquelette rigide agencée autour de ladite carcasse et solidarisée à ladite carcasse, la structure-exosquelette rigide comprenant une embase et plusieurs éléments longilignes solidaires de ladite embase et définissant un volume dans lequel vient se loger la carcasse en se positionnant sur l'embase, la majeure partie de la structure-exosquelette rigide étant espacée de ladite carcasse lorsque ladite carcasse est logée dans le volume, caractérisé en ce que la structure-exosquelette rigide comprend :
- quatre éléments longilignes, agencés le long de régions d'angle du ventilateur en étant espacés de celles-ci, lesdits quatre éléments longilignes se rejoignant dans une zone de jonction surmontant le ventilateur médical, et
- une poignée de portage située dans la zone de jonction des éléments longilignes, permettant à un utilisateur de saisir manuellement la structure-exosquelette et transporter l'ensemble ventilateur/structure-exosquelette.

Selon le mode de réalisation considéré, le ventilateur médical ou appareil d'assistance respiratoire de la technologie peut comprendre l'une ou plusieurs des caractéristiques suivantes :
- la structure-exosquelette englobe toute la carcasse du ventilateur, c'est-à-dire forme un carénage de protection rigide enveloppant/entourant toute (et uniquement) la carcasse du ventilateur.
- les éléments longilignes sont espacés les uns des autres.
- la structure-exosquelette rigide comprend une embase.
- les éléments longilignes sont solidaires de l'embase de la structure-exosquelette.
- l'embase de la structure-exosquelette est bombée vers l'extérieur.
- l'embase de la structure-exosquelette comprend une plaque légèrement recourbée.
- l'embase de la structure-exosquelette comprend une surface d'appui agencée sur la face extérieure de l'embase, de préférence la surface d'appui est bombée vers l'extérieur.
- la surface d'appui de l'embase de la structure-exosquelette repose sur et est au contact du sol (ou de toute autre surface), lorsque l'ensemble ventilateur/structure-exosquelette est posé sur le sol en position dite « debout » de manière à éviter que le ventilateur soit directement en contact avec le sol et à ainsi le protéger de dégradations éventuelles lorsque le sol est recouvert de sable, de boue, de neige, d'eau....
- les éléments longilignes ont préférentiellement une forme générale de bande ou de ruban.
- la majeure partie de la structure-exosquelette est espacée de la carcasse lorsque la carcasse est logée dans le volume de la structure-exosquelette, c'est-à-dire à l'exception de l'embase de la structure-exosquelette au niveau de laquelle vient se fixer la carcasse du ventilateur.
- la majeure partie de la structure-exosquelette est espacée de la carcasse d'une distance inférieure à 10 cm, de préférence inférieure à 5 cm, lorsque la carcasse est logée dans le volume de la structure-exosquelette.
- la carcasse vient se positionner sur l'embase de la structure-exosquelette rigide de manière à ce que la carcasse ne soit pas en contact direct avec la surface, tel le sol, sur laquelle le ventilateur est posé.
- la carcasse vient se fixer de manière détachable, i.e. amovible, sur l'embase de la structure-exosquelette.
- la carcasse est fixée à l'embase de la structure-exosquelette.
- la carcasse du ventilateur comprend une face avant ou façade portant un écran d'affichage et/ou une IHM.
- la structure-exosquelette, en particulier la surface d'appui de l'embase de la structure-exosquelette, est conformée de manière à ce que le ventilateur soit légèrement incliné, c'est-à-dire pas strictement vertical, lorsqu'il est posé en position « debout » sur une surface, notamment au sol, afin d'améliorer la visibilité de l'IHM pour l'utilisateur.
- la carcasse comprend une face arrière située à l'opposé de la face avant.
- la carcasse comprend deux faces latérales agencées entre la face avant et la face arrière, c'est-à-dire de part et d'autre de la carcasse, i.e. droite et gauche.
- la face avant, la face arrière et les deux faces latérales sont séparées par quatre régions d'angle faisant jonction entre les faces, c'est-à-dire qu'une région d'angle se trouve à la limite ou jonction entre deux faces successives, en particulier les régions d'angle sont des arêtes ou analogue.
- les ou des éléments longilignes de la structure-exosquelette sont positionnés, en tout ou en partie, sensiblement en regard, i.e. en face et à faible distance, des régions d'angle du ventilateur, c'est-à-dire les régions d'arête séparant les faces de la carcasse du ventilateur.
- la carcasse du ventilateur a une forme générale approximativement parallélépipédique.
- les éléments longilignes comprennent une première extrémité solidaire de l'embase.
- les éléments longilignes sont agencés de manière sensiblement verticale depuis l'embase lorsque le ventilateur est en position « debout », c'est-à-dire lorsqu'il repose sur l'embase de la structure-exosquelette.
- les éléments longilignes viennent de réunir et se solidariser, via une seconde extrémité, au niveau d'une zone de jonction de la structure-exosquelette surmontant le ventilateur.
- chaque élément longiligne comprend plusieurs portions ou tronçons successifs formant une structure générale non-rectiligne, c'est-à-dire que les tronçons sont séparés les uns des autres par des coudes (i.e. angles).
- chaque élément longiligne comprend plusieurs portions (ou tronçons) successives incluant une première portion portant la première extrémité, une deuxième portion portant la seconde extrémité et une portion intermédiaire située entre les première et deuxième portions.
- les première et deuxième portions et la portion intermédiaire sont rectilignes ou approximativement rectilignes.
- la première portion forme avec la portion intermédiaire un premier angle A supérieur à 90°.
- la seconde portion forme avec la portion intermédiaire un second angle B supérieur à 90°.
- les angles A et B sont comprise entre 110° et 150°, de préférence de l'ordre de 120 à 135°.
- les angles A et B sont égaux ou différents.
- la structure-exosquelette comprend quatre éléments longilignes qui sont sensiblement verticaux, lorsque le ventilateur est en position « debout », c'est-à-dire lorsque l'embase de la structure-exosquelette rigide repose sur une surface, tel le sol.
- les quatre éléments longilignes comprennent chacun un axe XX, les axes XX des quatre éléments longilignes étant (approximativement) parallèles entre eux.
- les axes XX des quatre éléments longilignes sont portés par leur portion intermédiaire.
- la structure-exosquelette est par ailleurs conformée pour que le ventilateur soit légèrement incliné (par rapport à la surface considérée) lorsqu'il repose en position « couchée » sur une surface, en particulier une surface horizontale ou horizontale, tel le sol, et que l'écran du ventilateur se retrouve alors au-dessus (i.e. vers le haut) et en position horizontale ou légèrement inclinée par rapport à la position horizontale.
- la structure-exosquelette comprend deux éléments longilignes dits « arrières » situés en regard de la face arrière de la carcasse du ventilateur, c'est-à-dire la face opposée à la façade avant de la carcasse portant l'écran.
- les deux éléments longilignes « arrières » comprennent des zones d'appui coplanaires, c'est-à-dire situées dans un même plan, de préférence situées dans leur portion intermédiaire d'axe AA, de manière à pouvoir poser l'ensemble ventilateur/structure-exosquelette en position dite « couchée » sur une surface, tel le sol, de sorte que l'écran du ventilateur se retrouve au-dessus et (approximativement) horizontalement ou légèrement incliné.
- les positions dite « couchée » et dite « debout » sont des positions stables de l'ensemble ventilateur/ structure-exosquelette. Autrement dit, la structure-exosquelette est conformée pour que le ventilateur puisse offrir plusieurs positions assurant une bonne stabilité, en particulier les positions dites "couchée" et "debout".
- les éléments longilignes se rejoignent deux à deux dans la zone de jonction.
- les éléments longilignes se rejoignent deux à deux de part et d'autre de la structure-exosquelette en formant deux structures de jonction en forme de « Y » ou de « V ».
- les deux structures de jonction en forme de « Y » ou de « V » sont situées de part et d'autre de la structure-exosquelette 3 et reliées entre elles par la région centrale comprenant la poignée de portage, préférentiellement la région centrale est de forme allongée.
- la région centrale de la zone de jonction forme la poignée de portage.
- la poignée de portage est dimensionnée pour permettre à un utilisateur, tel un secouriste, de la saisir manuellement et de soulever et transporter facilement l'ensemble ventilateur/structure-exosquelette à une main.
- la poignée de portage est prise en sandwich entre les deux structures de jonction en forme de « Y » ou de « V ».
- la structure-exosquelette comprend en outre des éléments de renforts additionnels, de préférence un ou des éléments de paroi intermédiaire, joignant deux éléments longilignes entre eux, par exemple à mi-distance des première et seconde extrémités desdits éléments longilignes, de manière à les fixer fermement l'un à l'autre et ainsi rigidifier davantage la structure-exosquelette.
- la structure-exosquelette comprend quatre éléments longilignes formant des paires, en particulier une paire d'éléments frontaux et une paire d'éléments arrières.
- les deux éléments longilignes d'une paire sont symétriques l'un de l'autre et situés à équidistance de la carcasse du ventilateur.
- la structure-exosquelette forme une cage rigide entourant (quasi)complètement la carcasse du ventilateur, c'est-à-dire englobant préférentiellement toutes les faces de la carcasse du ventilateur.
- la structure-exosquelette comprend des éléments longilignes se projetant vers le haut depuis l'embase portant le ventilateur, en entourant complètement ledit ventilateur mais en étant agencées à distance de celui-ci, i.e. de sa carcasse, et en se rejoignant au-dessus du ventilateur au niveau d'une zone de jonction formant poignée de portage, lorsque le ventilateur est en position debout.
- la carcasse contient une micro-soufflante.
- la micro-soufflante est équipée d'un moteur électrique entraînant en rotation un arbre, aussi appelé axe rotatif, portant une (ou des) roue à ailettes.
- la micro-soufflante comprend une volute délimitant un compartiment à roue dans lequel est agencée une roue à ailettes.
- la volute comprend un passage de sortie de gaz en communication fluidique avec le compartiment à roue, pour extraire dudit compartiment à roue, un flux gazeux généré par la roue à ailette, lorsque ladite roue à ailette est entraînée en rotation par l'arbre.
- la carcasse forme un boitier ou analogue.
- la carcasse comprend des parois rigides.
- la paroi avant formant toute ou partie de la façade avant comprend l'écran d'affichage.
- la carcasse est formée de polymère.
- la carcasse comprend en outre des moyens de pilotage configurés pour commander le fonctionnement ou l'arrêt du moteur électrique, c'est-à-dire les rotations et les arrêts de rotation (i.e. freinage ou décélération) de la roue de la micro-soufflante.
- les moyens de pilotage comprennent au moins un microprocesseur, de préférence au moins un microcontrôleur.
- les moyens de pilotage comprennent au moins une carte électronique portant ledit au moins un microprocesseur.
- le microprocesseur met en oeuvre un ou plusieurs algorithmes.
- la carcasse comprend des moyens d'alimentation électrique, en particulier une (ou des) batterie rechargeable.
- les moyens de pilotage sont alimentés en courant électrique par les moyens d'alimentation électrique.
- il comprend une IHM, c'est-à-dire une interface homme-machine.
- l'IHM comprend des moyens de sélection ou de réglage, telles des boutons ou touches.
- l'écran d'affichage est tactile et comprend, forme ou fait partie de l'IHM.
- le moteur électrique comprend des câbles ou fils électriques servant à son raccordement électrique à une source de courant électrique.
- durant son fonctionnement, le moteur électrique entraîne la roue à ailettes en rotation à une vitesse allant jusqu'à 70 000 tr/min, typiquement jusqu'à 30 000 ou 40 000 tr/min.
- le moteur électrique est de type sans balai (« brushless » en anglais).

L'invention va maintenant être mieux comprise grâce à la description détaillée suivante, faite à titre illustratif mais non limitatif, en référence aux figures annexées parmi lesquelles :
Fig.1 est une première vue, sensiblement de face, d'un mode de réalisation d'un ventilateur médical équipé d'une structure-exosquelette selon l'invention, montré en position dite « debout »,
Fig.2 est un mode de réalisation d'une structure-exosquelette pour un ventilateur médical selon l'invention,
Fig.3 est une vue de l'assemblage de la structure-exosquelette avec poignée sur la carcasse d'un ventilateur médical selon l'invention, en position intermédiaire, la structure-exosquelette avec poignée n'étant pas fixée à la carcasse,
Fig.4 est une vue en position inclinée du ventilateur de la Fig.1 ,
Fig.5 est une vue sensiblement latérale du ventilateur de la Fig.1 montré en position dite « couchée » et
Fig.6 est une vue de dessus de la partie arrière du ventilateur de la Fig.1.

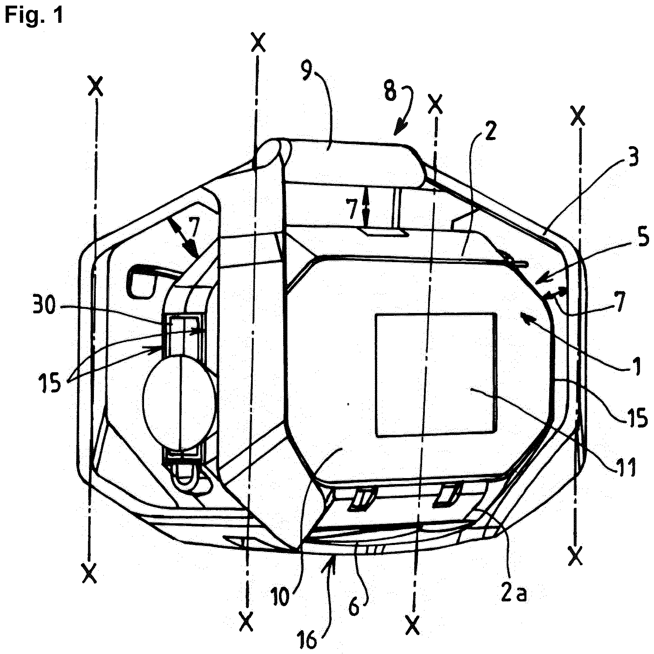

Fig.1 est une première vue, sensiblement de face, d'un mode de réalisation d'un ventilateur médical 1, typiquement d'urgence-transport, équipé d'une structure-exosquelette 3 selon l'invention agencée autour de la carcasse externe 1 ou boitier du ventilateur 1. La structure-exosquelette 3 protège uniquement le ventilateur 1, c'est-à-dire qu'elle ne vise pas à protéger un autre appareil ou dispositif additionnel éventuel associé au ventilateur 1.

Le ventilateur 1 est montré en position dite « debout », c'est-à-dire avec sa façade avant 10 portant l'écran d'affichage 11, sensiblement verticale et sa base 2a située au niveau de la surface (non montrée) sur laquelle il est posé, tel le sol.

L'écran d'affichage 11 permet de visualiser des informations, des données, des alarmes, des courbes de suivi etc... Il peut être en noir et blanc ou en couleurs. Il peut être tactile et comporter ou former une IHM, c'est-à-dire comporter des touches de sélection permettant d'opérer des réglages, des choix dans les menus, d'activer des fonctions, d'acquitter des alarmes, de sélectionner des modes ventilatoires ou d'autres paramètres...

La carcasse 2 du ventilateur 1 forme un boitier rigide, par exemple en polymère.

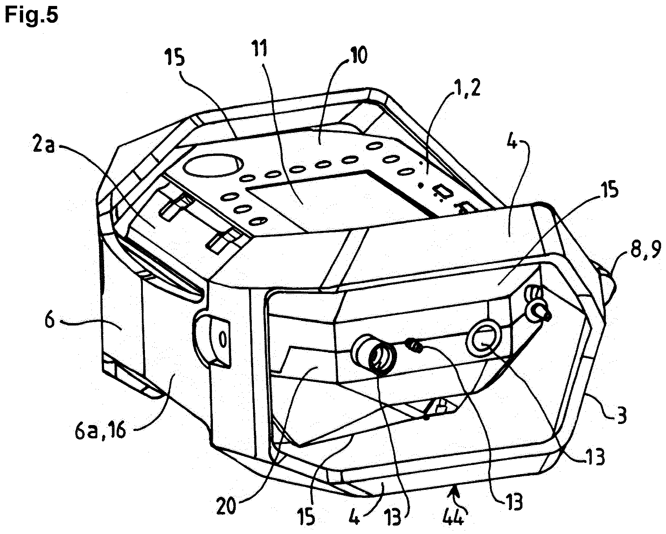

De manière classique, la carcasse 2 du ventilateur 1 porte différents éléments et composants, telles des prises, raccords ou embouts de raccordement 13 (cf. Fig. 5) auxquels viennent se raccorder mécaniquement et fluidiquement notamment des tuyaux ou autres conduits flexibles, notamment un circuit patient reliant le ventilateur 1 au patient et servant à acheminer le gaz jusqu'à lui, lequel comprend classiquement au moins un conduit flexible et se termine par une interface respiratoire, tel un masque respiratoire ou analogue.

La carcasse 2 du ventilateur 1 renferme par ailleurs des moyens de pilotage, telle une carte électronique à microprocesseur mettant en oeuvre un ou plusieurs algorithmes, lesquels sont configurés pour commander le fonctionnement ou l'arrêt du moteur électrique de la micro-soufflante, c'est-à-dire les rotations et les arrêts de rotation (i.e. freinage ou décélération) de la roue.

De préférence, le moteur électrique est de type sans balai et, durant son fonctionnement, il entraîne la roue à ailettes en rotation à une vitesse allant jusqu'à 70 000 tr/min, typiquement jusqu'à 30 000 ou 40 000 tr/min.

Il est aussi prévu des moyens d'alimentation électrique, en particulier une (ou des) batterie rechargeable, alimentant électriquement les moyens de pilotage, l'écran d'affichage, l'IHM, le moteur de la micro-soufflante ou d'autres composants du ventilateur qui requièrent du courant électrique pour fonctionner, notamment via des câbles ou fils électriques de raccordement électrique.

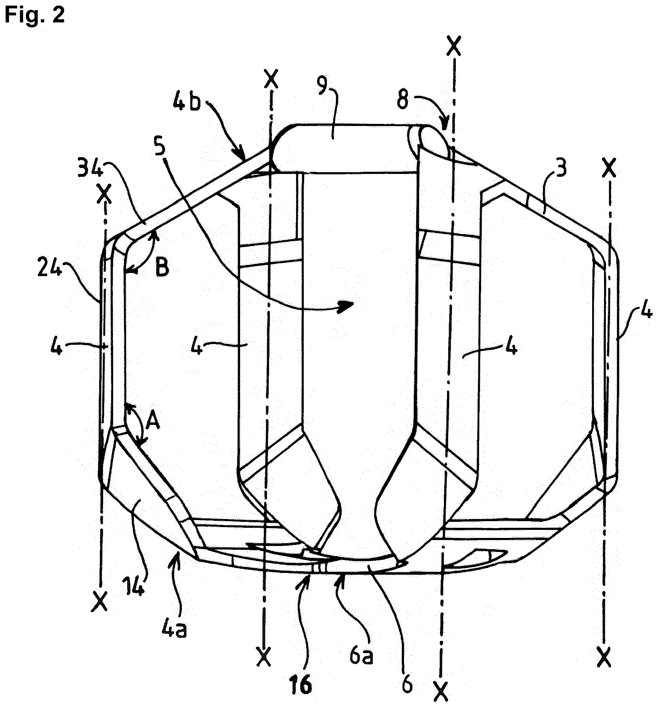

Selon la présente invention, le ventilateur médical 1 comprend une structure-exosquelette rigide 3 qui est agencée autour de la ladite carcasse 2 et est solidarisée à celle-ci. Un mode de réalisation de la structure-exosquelette rigide 3 est illustré en Fig. 2.

Cette structure-exosquelette rigide 3 peut être formée d'un ou plusieurs matériaux rigides, notamment en polymère, en métal ou alliage métallique, tel un alliage d'aluminium, de l'acier ou du Zamac, en matériau composite ou autre. L'ensemble de la structure-exosquelette rigide 3 peut être formée d'une seule pièce ou bien de plusieurs pièces ou sous-unités assemblées entre eux, par exemple par vissage ou autre.

Comme on le voit sur la Fig. 2, la structure-exosquelette rigide 3 comprend plusieurs éléments longilignes 4, aussi appelés « bras », espacés les uns des autres et conformés pour définir ou délimiter un volume 5, tel une cage ou capotage rigide, dans lequel vient se loger la carcasse 2 du ventilateur.

Dans ce mode de réalisation, la structure-exosquelette rigide 3 comprend une embase 6, ou socle, et quatre éléments longilignes 4 solidaires de ladite embase 6. Les éléments longilignes 4 peuvent être formés ou façonnés d'une seule pièce avec l'embase 6, par exemple par injection-moulage ou autre, ou bien y être fixées, directement ou indirectement, par exemple via une pièce intermédiaire. De préférence, l'embase 6 et les éléments longilignes 4 sont formés d'une pièce.

L'embase 6 a ici une forme de plaque légèrement recourbée ; toutefois, elle peut avoir une autre forme et/ou être ajourée. Prévoir une embase 6 légèrement recourbée, c'est-à-dire bombée vers l'extérieur, sur le dessous de la structure-exosquelette 3 est avantageux car cela permet, lors d'une chute éventuelle de l'ensemble ventilateur/ structure-exosquelette 1, 3, que l'énergie du choc ne soit pas intégralement transmise à l'appareil 1 mais soit partiellement transformée en énergie cinétique absorbée par la structure-exosquelette 3.

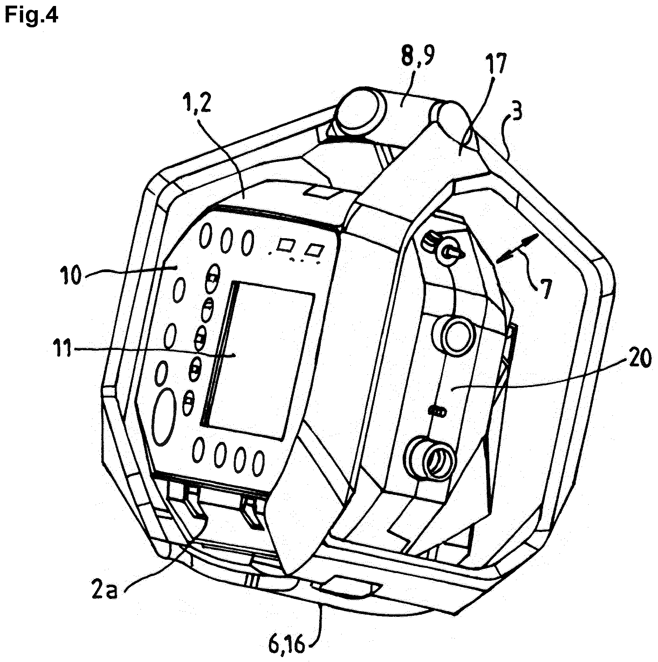

Cette embase 6 comprend une surface d'appui 16, sur sa face extérieure 6a qui est de préférence bombée extérieurement, venant reposer sur la surface, tel le sol, sur laquelle l'ensemble ventilateur 1/ structure-exosquelette 3 est posée en position dite « debout », comme illustré sur les Fig. 1 à Fig. 4. Ceci permet d'éviter que le ventilateur 1 soit directement en contact avec le sol, ce qui permet de le protéger de dégradations éventuelles lorsque le sol est recouvert de sable, de boue, de neige, d'eau....

Les éléments longilignes 4 ont, quant à eux, ici une forme générale de bande ou de ruban mais, là encore, ils pourraient avoir une autre forme, par exemple une forme tubulaire, notamment cylindrique ou analogue. On peut aussi prévoir plus de quatre éléments longilignes 4 et/ou ceux-ci peuvent être divisés longitudinalement. En outre, les éléments longilignes 4 n'ont pas obligatoirement tous la même forme et/ou les mêmes dimensions.

Comme illustré, les éléments longilignes 4 se projettent par ailleurs vers le haut et viennent se réunir au-dessus du ventilateur 1, c'est-à-dire dans une zone de jonction 8 située au-dessus du sommet 2b de la carcasse 2, c'est-à-dire à l'opposé de la base 2a.

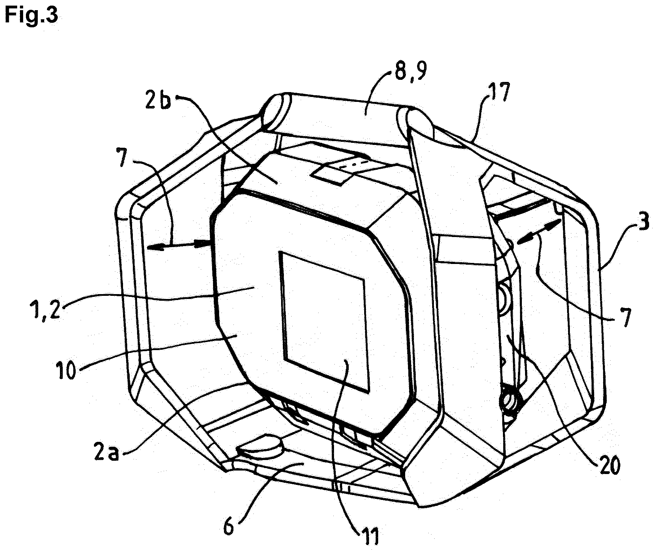

Plus précisément, comme on le voit sur les Fig. 3, Fig. 4 et Fig. 6, dans la zone de jonction 8, les éléments longilignes 4 se rejoignent deux à deux, de part et d'autre de la structure-exosquelette 3, en formant deux structures de jonction 17 en forme de « Y » ou de « V » situées, elles aussi, de part et d'autre de la structure-exosquelette 3 et reliées entre elles par une région centrale, préférentiellement de forme allongée.

Avantageusement, la région centrale de la zone de jonction 8 forme une poignée 9 de portage dimensionnée pour permettre à un utilisateur, tel un secouriste, de la saisir manuellement, c'est-à-dire à une main, et de soulever et transporter facilement l'ensemble ventilateur 1/ structure-exosquelette 3. Autrement dit, la poignée 9 de portage est prise en sandwich entre les deux structures de jonction 17 en forme de « Y » ou de « V ».

La poignée 9 de portage a par exemple une forme cylindrique et est agencée horizontalement lorsque le ventilateur 1 est posé en position dite « debout » sur une surface, telle sol, comme montré par exemple en Fig. 1 et Fig. 3. La poignée 9 de portage peut être formée ou façonnée d'une seule pièce avec le reste de la structure-exosquelette 3 ou bien être fixée, directement ou indirectement, aux éléments longilignes 4. Elle peut être formé d'une ou plusieurs pièces. Elle peut être recouverte d'un matériau souple ou « mou au toucher », par exemple un élastomère ou de la silicone, pour améliorer la sensation pour l'utilisateur qui la prend en main, de préférence un matériau souple de dureté Shore de 0 à 50.

De préférence, la poignée 9 de portage est agencée sensiblement parallèlement à la plaque formant toute ou partie de l'embase 6 comme schématisé en Fig. 2. Toutefois, il faut noter que la poignée 9 est optionnelle, c'est-à-dire qu'elle n'est pas toujours indispensable et peut donc être supprimée dans certains cas d'utilisation. En outre, selon le mode de réalisation choisi, on peut aussi prévoir plusieurs poignée 9 de portage agencées sur la structure-exosquelette rigide 3;

Il est à souligner qu'un utilisateur peut aussi se saisir de l'ensemble et le transporter via une préhension d'un des éléments longilignes 4, c'est-à-dire en lieu et place d'une préhension de la poignée 9 de portage.

Plus généralement, la structure-exosquelette rigide 3 présente un ou plusieurs plans de symétrie, en particulier la partie droite de la structure-exosquelette 3 illustrée en Fig. 2 est (approximativement) symétrique de sa partie gauche, et/ou sa partie avant est également (approximativement) symétrique de sa partie arrière.

Par ailleurs, lorsque la carcasse 2 du ventilateur 1 est logée dans le volume 5 qui est défini ou délimité entre les éléments longilignes 4, l'embase 6 et la zone de jonction 8 incluant la poignée 9, la majeure partie de la structure-exosquelette rigide 3 est espacée 7 de la carcasse 2, c'est-à-dire que les éléments longilignes 4 ne sont pas en contact direct avec la surface/paroi périphérique de la carcasse 2, sauf au niveau de l'embase 6, mais en sont espacés, par exemple de quelques mm à quelques cm, de sorte qu'un choc mécanique ou une chute entraînant une déformation de la structure-exosquelette 3, c'est-à-dire d'un (ou plusieurs) élément longiligne 4 puisse être absorbé par la structure-exosquelette 3 et ne pas se répercuter sur la paroi périphérique de la carcasse 2 du ventilateur 1 et ainsi le protéger efficacement.

Plus précisément, la carcasse 2 du ventilateur 1 vient reposer ici sur l'embase 6 de la structure-exosquelette rigide 3 et est fixée, via sa base 2a, au niveau de ladite embase 6, ce qui permet par ailleurs d'isoler le ventilateur 1 du sol et ainsi éviter qu'il ne soit en contact direct avec des milieux pouvant le détériorer, tel que sable, terre, graviers, neige, humidité... puisque c'est alors la structure-exosquelette 3 qui est en contact avec le sol via son embase 6.

De plus, fixer la structure-exosquelette rigide 3 à la carcasse 2 du ventilateur dans sa partie basse, c'est-à-dire au niveau de sa base 2a, en espaçant 7 la majeure partie de la structure-exosquelette rigide 3 de la carcasse 2, permet de laisser place à une déformation de la structure-exosquelette rigide 3 en cas de chute/choc impactant les éléments longilignes 4 ou d'autres parties de la structure-exosquelette, mais sans impacter la carcasse 2 du ventilateur 1 elle-même et donc sans nuire au bon fonctionnement du ventilateur 1. Bien entendu, la structure-exosquelette 3 peut aussi venir se solidariser à la carcasse 2 du ventilateur 1 à un ou plusieurs autres endroits, c'est-à-dire ailleurs qu'au niveau de sa base 2a, selon le mode de réalisation choisi.

Plus précisément, les éléments longilignes 4 comprennent chacun une première extrémité 4a solidaire de l'embase 6 et une seconde extrémité 4b solidaire de la poignée 9 de portage, c'est-à-dire au niveau de la zone de jonction 8, c'est-à-dire que les éléments longilignes 4 sont agencés et s'étirent de manière sensiblement verticale lorsque le ventilateur 1 est en position « debout ».

Comme on le voit sur la Fig. 2, dans le mode de réalisation présenté, chaque élément longiligne 4 comprend plusieurs portions successives incluant une première portion 14 portant la première extrémité 4a, une deuxième portion 34 portant la seconde extrémité 4b et une portion intermédiaire 24 prise « en sandwich » entre les première et deuxième portions 14, 34, c'est-à-dire située entre celles-ci.

De préférence, les première et deuxième portions 14, 34 et la portion intermédiaire 24 sont rectilignes ou approximativement rectilignes.

La première portion 14 forme avec la portion intermédiaire 24 un premier angle A supérieur à 90°, et la seconde portion 34 forme avec la portion intermédiaire 24 un second angle B supérieur à 90°, lesdits angles A, B étant égaux ou différents, par exemple des angles de l'ordre de 110 à 150°, par exemple de l'ordre de 120 à 135°.

Par ailleurs, comme illustré en Fig. 1 et Fig. 2, la structure-exosquelette 3 est préférentiellement conformée pour présenter quatre éléments longilignes 4 sensiblement verticaux, lorsque le ventilateur 1 est en position « debout ».

Avantageusement, les quatre éléments longilignes 4 comprennent chacun un axe XX, qui est par exemple porté par leur portion intermédiaire 24, les différents axes XX étant parallèles entre eux.

De préférence, la structure-exosquelette 3 comprend deux éléments longilignes 4 dits « arrières » situés en regard de la face arrière 40 du ventilateur 1, c'est-à-dire la face opposée à la façade avant 10 portant l'écran 11, lesdits deux éléments longilignes 4 « arrières » comprenant des zones d'appui 44 coplanaires, par exemple situées dans leur portion intermédiaire 24 d'axe AA, de manière à pouvoir poser l'ensemble ventilateur 1/structure-exosquelette 3 en position dite « couchée » sur une surface, tel le sol, comme illustré en Fig. 5, de sorte que l'écran 11 du ventilateur 1 se retrouve au-dessus, c'est-à-dire sensiblement horizontalement par rapport à ladite surface, tel le sol ou analogue.

Il est à noter que la structure-exosquelette 3 peut aussi comprendre des éléments de renforts additionnels (non montré), par exemple un (des) élément de paroi intermédiaire joignant deux éléments longilignes 4 entre eux, par exemple à mi-distance des première et seconde extrémités 4a, 4b, de manière à les fixer fermement l'un à l'autre et ainsi rigidifier davantage la structure-exosquelette 3.

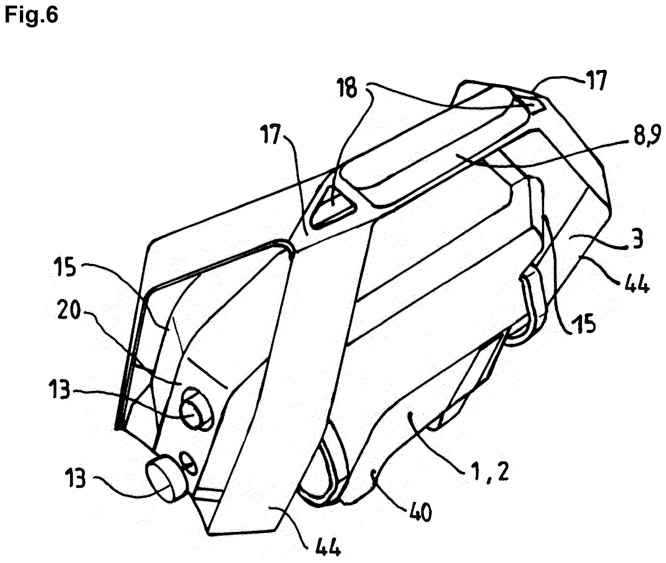

Comme visible sur Fig. 1 et Fig. 3 à Fig. 5, la carcasse 2 du ventilateur 1 comprend une face avant ou façade 10 portant l'écran 11, une face arrière 40 (cf. Fig. 6) et deux faces latérales droite 20 et gauche 30 agencées entre la façade 10 et la face arrière 40, de part et d'autre de la carcasse 2. Ces quatre faces 10, 20, 30, 40 successives sont séparées par quatre régions d'angle 15 faisant jonction entre les différentes faces 10, 20, 30, 40.

Avantageusement, les éléments longilignes 4 de la structure-exosquelette 3 viennent se positionner, en tout ou en partie, sensiblement en regard, i.e. en face et à distance, des régions d'angle 15, telles des arêtes, séparant la façade 10 et la face arrière 40 de la carcasse 2 des deux faces latérales droite 20 et gauche 30.

D'une façon générale, la structure-exosquelette 3, en particulier l'embase 6 et les éléments longilignes 4, en particulier les deux éléments longilignes 4 « arrières » présentant les zones d'appui 44 coplanaires, est conformée pour assurer une bonne stabilité verticale et horizontale du ventilateur 1 lorsqu'il est posé que ce soit en position « debout » comme montré en Fig. 3, ou en position « couchée » comme montré en Fig. 5.

Par ailleurs, comme visible sur les Fig. 1 à Fig. 3 notamment, la structure-exosquelette 3 comprend quatre éléments longilignes agencés en paires, en particulier une paire d'éléments longilignes frontaux situés du côté de la face avant 10 du ventilateur 1, et la paire d'éléments longilignes arrières situés en regard de la face arrière 40 du ventilateur 1.

Avantageusement, les deux éléments longilignes 4 d'une paire donnée sont symétriques l'un par rapport à l'autre et sont en outre situés à équidistance de la carcasse du ventilateur 1. Ainsi, l'élément longiligne frontal droit est symétrique de l'élément longiligne frontal gauche et, de même, l'élément longiligne arrière droit est symétrique de l'élément longiligne arrière gauche. De plus, les deux éléments longilignes 4 de deux paires différentes peuvent aussi être symétriques les uns aux autres.

Selon un mode de réalisation particulier, on peut conformer la structure-exosquelette 3, notamment la surface d'appui 16 de son embase 6, de manière à ce que le ventilateur 1 soit légèrement incliné, et non pas strictement vertical, lorsqu'il est posé en position « debout » sur une surface, notamment au sol, de manière à améliorer la visibilité de l'IHM, en particulier de l'écran 11, des touches ou boutons de sélection ou autres, ...), pour l'utilisateur.

Plus généralement, on veille à ce que la structure-exosquelette 3 qui constitue à la fois une structure de préhension et de protection située autour du ventilateur 1, soit agencée de telle sorte qu'aucun élément du ventilateur ne dépasse, c'est-à-dire ne fasse saillie, au-delà de celle-ci, c'est-à-dire au-delà du volume interne qu'elle délimite et dans lequel le ventilateur 1 est inséré.

Comme déjà dit, la structure-exosquelette 3 permet une préhension multiple par l'utilisateur de l'ensemble ventilateur 1/ structure-exosquelette 3, via la poignée principale 9 se situant sur le dessus du ventilateur 1 et via les éléments longilignes 4, ce qui facilite la préhension de l'ensemble 1,3 dans des situations diverses. La poignée 9 est agencé à une distance 7 suffisante de la carcasse 2 du ventilateur 1 pour laisser un espacement suffisant pour permettre sa prise en main même lorsque l'utilisateur, tel un secouriste, porte des gants de protection, par exemple des gants de montagne ou des gants de pompiers.

En outre, selon un mode de réalisation particulier, on peut prévoir des surmoulages d'élastomère ou analogues sur la structure-exosquelette 3 afin d'améliorer la résistance aux chutes, par exemple dans sa partie basse 6.

Comme illustré en Fig. 6, la structure-exosquelette 3 peut comprendre des moyens d'arrimage 18, telles des ouvertures, des boucles, des crochets ou autres, configurés pour recevoir des mousquetons ou autres moyens d'accroche complémentaires, pour permettre une fixation de l'ensemble ventilateur 1/ structure-exosquelette 3 dans un véhicule, par exemple une ambulance, un hélicoptère ou autre, ou sur un brancard, un lit ou autre.

Dans le mode de réalisation présente en Fig. 6, les moyens d'arrimage 18 sont des ouvertures aménagées dans la zone de jonction 8 où les éléments longilignes 4 se rejoignent deux à deux, de part et d'autre de la structure-exosquelette 3, en formant les deux structures de jonction 17 en forme de « Y » ou de « V » situées de part et d'autre de la région centrale formant la poignée 9 de préhension et portage.

D'une façon générale, la structure-exosquelette 3 de l'invention constitue une armature externe rigide du ventilateur 1 cumulant plusieurs fonctions et présentant de nombreux avantages, notamment :
- protéger le ventilateur 1 contre les chutes et les chocs, donc de rendre le ventilateur 1 plus robuste et fiable.
- permettre une préhension multiple de l'ensemble grâce à la poignée de préhension principale située en regard de la face supérieure ou sommet du ventilateur médical et aux éléments longilignes 4 formant des zones de préhension alternatives situées en regard des faces latérales du ventilateur 1.
- permettre une bonne visibilité de l'IHM lorsque l'appareil est posé au sol, malgré la présence de la structure-exosquelette 3.
- assurer la stabilité verticale et horizontale du ventilateur 1 lorsqu'il est posé au sol ou sur une autre surface, telle une table, le fond d'un véhicule ou autre.
- protéger le ventilateur 1 de la surface du sol en évitant un contact direct entre eux, notamment lorsque le sol est recouvert ou formé de sable, de neige, de terre, d'eau...
- assurer un bon refroidissement du ventilateur médical en le surélevant du sol.

## Revendications

1. Ventilateur médical (1) comprenant une carcasse externe (2), et une structure-exosquelette rigide (3) agencée autour de ladite carcasse (2) et solidarisée à ladite carcasse (2), la structure-exosquelette rigide (3) comprenant une embase (6) et plusieurs éléments longilignes (4) solidaires de ladite embase (6) et définissant un volume (5) dans lequel vient se loger la carcasse (2) en se positionnant sur l'embase (6), la majeure partie de la structure-exosquelette rigide (3) étant espacée (7) de ladite carcasse (2) lorsque ladite carcasse (2) est logée dans le volume (5), **caractérisé en ce que** la structure-exosquelette rigide (3) comprend :
- quatre éléments longilignes (4), agencés le long de régions d'angle (15) du ventilateur (1) en étant espacés de celles-ci, lesdits quatre éléments longilignes (4) se rejoignant dans une zone de jonction (8) surmontant le ventilateur médical (1), et
- une poignée de portage (9) située dans la zone de jonction (8) des éléments longilignes (4), permettant à un utilisateur de saisir manuellement la structure-exosquelette (3) et transporter l'ensemble (1, 3) ventilateur/structure-exosquelette.

2. Ventilateur médical selon la revendication 1, **caractérisé en ce que** les éléments longilignes (4) sont agencés de manière sensiblement verticale depuis l'embase (6) lorsque le ventilateur (1) est en position « debout » et repose sur l'embase (6) de la structure-exosquelette (3).

3. Ventilateur médical selon la revendication 2, **caractérisé en ce que** les éléments longilignes (4) comprennent une première extrémité solidaire de l'embase (6) et viennent de réunir et se solidariser, via une seconde extrémité, au niveau d'une zone de jonction (8) de la structure-exosquelette (3) surmontant le ventilateur (1).

4. Ventilateur médical selon la revendication 1, **caractérisé en ce que** l'embase (6) de la structure-exosquelette (3) comprend une surface d'appui (16) et/ou est bombée vers l'extérieur.

5. Ventilateur médical selon la revendication 1, **caractérisé en ce que** les éléments longilignes (4) ont une forme générale de bande ou de ruban.

6. Ventilateur médical selon la revendication 1, **caractérisé en ce que**, dans la zone de jonction (8), les éléments longilignes (4) se rejoignent deux à deux en formant deux structures de jonction (17) en forme de « Y » ou de « V » situées de part et d'autre de la structure-exosquelette (3) et reliées entre elles par une région centrale formant la poignée (9) de portage.

7. Ventilateur médical selon l'une des revendications précédentes, **caractérisé en ce que** la structure-exosquelette (3) comprend deux éléments longilignes arrières (4) situés en regard de la face arrière (40) de la carcasse (2) du ventilateur (1), les deux éléments longilignes arrières (4) comprenant des zones d'appui (44) coplanaires.

8. Ventilateur médical selon l'une des revendications précédentes, **caractérisé en ce que** la carcasse (2) du ventilateur (1) comprend une face avant (10) portant un écran d'affichage (11) et/ou une IHM, une face arrière (40) située à l'opposé de la face avant (10), et deux faces latérales (20, 30) agencées entre la face avant (10) et la face arrière (40), lesdites face avant, face arrière et deux faces latérales étant séparées par quatre régions d'angle (15) faisant jonction entre les faces (10, 20, 30, 40), et les ou des éléments longilignes (4) de la structure-exosquelette (3) étant au moins en partie positionnés en regard des régions d'angle (15) séparant lesdites faces (10, 20, 30, 40).

9. Ventilateur médical selon la revendication 1, **caractérisé en ce que** la structure-exosquelette (3) forme une cage rigide entourant complètement la carcasse (2) du ventilateur (1).

10. Ventilateur médical selon la revendication 1, **caractérisé en ce que** chaque élément longiligne (4) comprend plusieurs portions successives incluant une première portion portant la première extrémité, une deuxième portion portant la seconde extrémité et une portion intermédiaire située entre les première et deuxième portions, la première portion formant avec la portion intermédiaire un premier angle A supérieur à 90° et la seconde portion formant avec la portion intermédiaire un second angle B supérieur à 90°.

11. Ventilateur médical selon la revendication 1, **caractérisé en ce que** la majeure partie de la structure-exosquelette (3) est espacée de la carcasse (2) d'une distance inférieure à 10 cm, de préférence inférieure à 5 cm.

12. Ventilateur médical selon la revendication 1, **caractérisé en ce que** la structure-exosquelette (3) est conformée pour que le ventilateur (1) soit légèrement incliné par rapport à la verticale, lorsqu'il repose en position « debout » sur une surface, notamment le sol.

13. Ventilateur médical selon la revendication 12, **caractérisé en ce que** la surface d'appui (16) de l'embase (6) est conformée pour que le ventilateur 1 soit légèrement incliné lorsqu'il est en position « debout ».

14. Ventilateur médical selon la revendication 1, **caractérisé en ce que** la structure-exosquelette (3) est par ailleurs conformée pour que le ventilateur (1) soit légèrement incliné lorsqu'il repose en position « couchée » sur une surface, et que l'écran (11) du ventilateur (1) se retrouve au-dessus et en position horizontale ou légèrement inclinée par rapport à la position horizontale.

## Patentansprüche

1. Medizinisches Beatmungsgerät (1), umfassend ein äußeres Gehäuse (2) und eine starre Exoskelett-Struktur (3), die um das Gehäuse (2) herum angeordnet ist und mit dem Gehäuse (2) verbunden ist, wobei die starre Exoskelett-Struktur (3) eine Basis (6) und mehrere langgliedrige Elemente (4), die mit der Basis (6) fest verbunden sind, umfasst und ein Volumen (5) definiert, in dem das Gehäuse (2) aufgenommen wird, indem es sich auf der Basis (6) positioniert, wobei der überwiegende Teil der starren Exoskelett-Struktur (3) von dem Gehäuse (2) beabstandet ist (7), wenn das Gehäuse (2) in dem Volumen (5) aufgenommen ist, **dadurch gekennzeichnet, dass** die starre Exoskelett-Struktur (3) umfasst:
- vier langgliedrige Elemente (4), die entlang von Eckregionen (15) des Beatmungsgeräts (1) angeordnet sind, wobei sie von diesen beabstandet sind, wobei sich die vier langgliedrigen Elemente (4) in einem Verbindungsbereich (8) treffen, der über dem medizinischen Beatmungsgerät (1) liegt, und
- einen Tragegriff (9), der in dem Verbindungsbereich (8) der langgliedrigen Elemente (4) gelegen ist und es einem Benutzer ermöglicht, die Exoskelett-Struktur (3) mit der Hand zu ergreifen und die Einheit (1, 3) Beatmungsgerät/Exoskelett-Struktur zu transportieren.

2. Medizinisches Beatmungsgerät nach Anspruch 1, **dadurch gekennzeichnet, dass** die langgliedrigen Elemente (4) im Wesentlichen vertikal von der Basis (6) aus angeordnet sind, wenn das Beatmungsgerät (1) in "aufrechter" Position ist und auf der Basis (6) der Exoskelett-Struktur (3) aufliegt.

3. Medizinisches Beatmungsgerät nach Anspruch 2, **dadurch gekennzeichnet, dass** die langgliedrigen Elemente (4) ein mit der Basis (6) fest verbundenes erstes Ende umfassen und über ein zweites Ende an einem Verbindungsbereich (8) der Exoskelett-Struktur (3), die über dem Beatmungsgerät (1) liegt, zusammengeführt werden und verbunden sind.

4. Medizinisches Beatmungsgerät nach Anspruch 1, **dadurch gekennzeichnet, dass** die Basis (6) der Exoskelett-Struktur (3) eine Auflagefläche (16) umfasst und/oder nach außen hin gewölbt ist.

5. Medizinisches Beatmungsgerät nach Anspruch 1, **dadurch gekennzeichnet, dass** die langgliedrigen Elemente (4) die Grundform eines Bands oder Streifens haben.

6. Medizinisches Beatmungsgerät nach Anspruch 1, **dadurch gekennzeichnet, dass** sich die langgliedrigen Elemente (4) in dem Verbindungsbereich (8) paarweise treffen, indem sie zwei Y- oder V-förmige Verbindungsstrukturen (17) bilden, die beidseits der Exoskelett-Struktur (3) gelegen sind und untereinander durch eine zentrale Region, die den Tragegriff (9) bildet, verbunden sind.

7. Medizinisches Beatmungsgerät nach einem der vorhergehenden Ansprüche, **dadurch gekennzeichnet, dass** die Exoskelett-Struktur (2) zwei hintere langgliedrige Elemente (4) umfasst, die gegenüber der Rückseite (40) des Gehäuses (2) des Beatmungsgeräts (1) gelegen sind, wobei die beiden hinteren langgliedrigen Elemente (4) komplanare Auflagebereiche (44) umfassen.

8. Medizinisches Beatmungsgerät nach einem der vorhergehenden Ansprüche, **dadurch gekennzeichnet, dass** das Gehäuse (2) des Beatmungsgeräts (1) eine Vorderseite (10), die einen Anzeigebildschirm (11) und/oder ein MMI trägt, eine Rückseite (40), die entgegengesetzt zu der Vorderseite (10) gelegen ist, und zwei seitliche Seiten (20, 30), die zwischen der Vorderseite (10) und der Rückseite (40) angeordnet sind, umfasst, wobei die Vorderseite, die Rückseite und die beiden seitlichen Seiten durch vier Eckregionen (15) getrennt sind, die einen Übergang zwischen den Seiten (10, 20, 30, 40) bilden, und wobei die oder einige der langgliedrigen Elemente (4) der Exoskelett-Struktur (3) mindestens zum Teil gegenüber den Eckregionen (15), welche die Seiten (10, 20, 30, 40) trennen, positioniert sind.

9. Medizinisches Beatmungsgerät nach Anspruch 1, **dadurch gekennzeichnet, dass** die Exoskelett-Struktur (3) einen starren Käfig bildet, der das Gehäuse (2) des Beatmungsgeräts (1) vollständig umgibt.

10. Medizinisches Beatmungsgerät nach Anspruch 1, **dadurch gekennzeichnet, dass** jedes langgliedrige Element (4) mehrere aufeinander folgende Abschnitte umfasst, die einen ersten Abschnitt, der das erste Ende trägt, einen zweiten Abschnitt, der das zweite Ende trägt, und einen Zwischenabschnitt, der zwischen den ersten und zweiten Abschnitten gelegen ist, beinhalten, wobei der erste Abschnitt mit dem Zwischenabschnitt einen ersten Winkel A von mehr als 90° bildet und wobei der zweite Abschnitt mit dem Zwischenabschnitt einen zweiten Winkel B von mehr als 90° bildet.

11. Medizinisches Beatmungsgerät nach Anspruch 1, **dadurch gekennzeichnet, dass** der überwiegende Teil der Exoskelett-Struktur (3) von dem Gehäuse um einen Abstand von weniger als 10 cm, bevorzugt weniger als 5 cm, beabstandet ist.

12. Medizinisches Beatmungsgerät nach Anspruch 1, **dadurch gekennzeichnet, dass** die Exoskelett-Struktur (3) in Bezug auf die Vertikale leicht geneigt ist, wenn sie in der "aufrechten" Position auf einer Fläche, insbesondere dem Boden, aufliegt.

13. Medizinisches Beatmungsgerät nach Anspruch 12, **dadurch gekennzeichnet, dass** die Auflagefläche (16) des Sockels (6) so geformt ist, dass das Beatmungsgerät 1 leicht geneigt ist, wenn es in der "aufrechten" Position ist.

14. Medizinisches Beatmungsgerät nach Anspruch 1, **dadurch gekennzeichnet, dass** die Exoskelett-Struktur (3) im Übrigen so geformt ist, dass das Beatmungsgerät (1) leicht geneigt ist, wenn es in "liegender" Position auf einer Fläche aufliegt, und dass der Bildschirm (11) des Beatmungsgeräts oben und in horizontaler Position oder in Bezug auf die horizontale Position leicht geneigt ist.

## Claims

1. Medical ventilator (1) comprising an outer shell (2), and a rigid exoskeleton structure (3) arranged around said shell (2) and rigidly connected to said shell (2), the rigid exoskeleton structure (3) comprising a base (6) and a plurality of elongate elements (4) rigidly connected to said base (6) and defining a volume (5) in which the shell (2) is housed by positioning itself on the base (6), the greater part of the rigid exoskeleton structure (3) being spaced (7) from said shell (2) when said shell (2) is housed in the volume (5), **characterized in that** the rigid exoskeleton structure (3) comprises:
- four elongate elements (4), arranged along corner regions (15) of the ventilator (1) and spaced apart from said regions, said four elongate elements (4) joining each other in a joining zone (8) above the medical ventilator (1), and
- a carrying handle (9) situated in the joining zone (8) of the elongate elements (4), allowing a user to manually grasp the exoskeleton structure (3) and transport the assembly (1, 3) composed of ventilator and exoskeleton structure.

2. Medical ventilator according to Claim 1, **characterized in that** the elongate elements (4) are arranged substantially vertically from the base (6) when the ventilator (1) is in an "upright" position and resting on the base (6) of the exoskeleton structure (3).

3. Medical ventilator according to Claim 2, **characterized in that** the elongate elements (4) comprise a first end rigidly connected to the base (6) and meet and join each other, via a second end, at a joining zone (8) of the exoskeleton structure (3) above the ventilator (1) .

4. Medical ventilator according to Claim 1, **characterized in that** the base (6) of the exoskeleton structure (3) comprises a bearing surface (16) and/or is curved outwards.

5. Medical ventilator according to Claim 1, **characterized in that** the elongate elements (4) have the general shape of a band or tape.

6. Medical ventilator according to Claim 1, **characterized in that**, in the joining zone (8), the elongate elements (4) join each other in pairs, forming two Y-shaped or V-shaped joining structures (17) which are situated on either side of the exoskeleton structure (3) and are connected to each other by a central region that forms the carrying handle (9).

7. Medical ventilator according to one of the preceding claims, **characterized in that** the exoskeleton structure (3) comprises two rear elongate elements (4) situated opposite the rear face (40) of the shell (2) of the ventilator (1), the two rear elongate elements (4) comprising coplanar bearing zones (44).

8. Medical ventilator according to one of the preceding claims, **characterized in that** the shell (2) of the ventilator (1) comprises a front face (10) supporting a display screen (11) and/or a HMI, a rear face (40) situated opposite the front face (10), and two lateral faces (20, 30) arranged between the front face (10) and the rear face (40), said front face, rear face and two lateral faces being separated by four corner regions (15) forming a join between the faces (10, 20, 30, 40), and the one or more elongate elements (4) of the exoskeleton structure (3) being at least in part positioned opposite the corner regions (15) separating said faces (10, 20, 30, 40).

9. Medical ventilator according to Claim 1, **characterized in that** the exoskeleton structure (3) forms a rigid cage that completely surrounds the shell (2) of the ventilator (1).

10. Medical ventilator according to Claim 1, **characterized in that** each elongate element (4) comprises a plurality of successive portions, including a first portion having the first end, a second portion having the second end, and an intermediate portion situated between the first and second portions, the first portion forming a first angle A of greater than 90° with the intermediate portion, and the second portion forming a second angle B of greater than 90° with the intermediate portion.

11. Medical ventilator according to Claim 1, **characterized in that** the greater part of the exoskeleton structure (3) is spaced from the shell (2) by a distance of less than 10 cm, preferably less than 5 cm.

12. Medical ventilator according to Claim 1, **characterized in that** the exoskeleton structure (3) is configured such that the ventilator (1) is slightly inclined with respect to the vertical when it rests in an "upright" position on a surface, particularly the ground.

13. Medical ventilator according to Claim 12, **characterized in that** the bearing surface (16) of the base (6) is configured such that the ventilator 1 is slightly inclined when it is in the "upright" position.

14. Medical ventilator according to Claim 1, **characterized in that** the exoskeleton structure (3) is moreover configured such that the ventilator (1) is slightly inclined when it rests in a "recumbent" position on a surface, and **in that** the screen (11) of the ventilator (1) is located at the top and in a horizontal position or in a position slightly inclined with respect to the horizontal position.
